# EUROPEAN PATENT SPECIFICATION

(11) **EP 3 970 649 B1**
(45) Date of publication and mention of the grant of the patent: **28.08.2024**
(21) Application number: 21197218.7
(22) Date of filing: 16.09.2021
(51) Int. Cl.: A61B 18/14

(54) **RF ABLATION CATHETER FOR TREATMENT OF ATRIAL FIBRILLATION**
HF-ABLATIONSKATHETER ZUR BEHANDLUNG VON VORHOFFLIMMERN
CATHÉTER D'ABLATION RF POUR LE TRAITEMENT DE LA FIBRILLATION AURICULAIRE

(30) Priority: 18.09.2020 KR 20200120930
(43) Date of publication of application: 23.03.2022
(73) Proprietor: Tau Medical Inc., Busan (KR)
(72) Inventor: KIM, June Hong, 48516 Busan (KR); PARK, Kyone, 50653 Yangsan (KR); WON, Yong-Hyun, 50653 Yangsan-si (KR); CHO, Min Soo, 124403 Gunpo-si (KR)
(74) Representative: dompatent von Kreisler Selting Werner - Partnerschaft von Patent- und Rechtsanwälten mbB

(56) References cited:
- WO-A1-2019/148094
- US-A1- 2016 128 767
- US-A1- 2019 076 179
- US-A1- 2020 155 229
- US-B1- 6 251 107
- US-B2- 8 834 464
- US-B2- 8 998 894

## Description

### BACKGROUND

### 1. Field of the Invention

The present disclosure relates to a RF ablation catheter for treatment of atrial fibrillation and a method for treatment of atrial fibrillation using the same, and more particularly, to a RF ablation catheter for treatment of atrial fibrillation and a method for treatment of atrial fibrillation using the same capable of, in order to treat atrial fibrillation caused in the electrical system of the heart, eliminating an abnormal electrical signal, which is generated in the vicinity of the vein of Marshall, using radio frequency ablation (RFA) to eliminate atrial fibrillation.

### 2. Discussion of Related Art

The heart consists of two atria and two ventricles. It is normal for blood to sequentially move from the atria to the ventricles and from the ventricles to the lungs or the whole body by regular contraction and expansion of the atria and ventricles. "Atrial fibrillation" is a type of arrhythmic (irregular pulse) disease in which atria do not beat regularly and various parts of the atria beat chaotically, resulting in an extremely-high-speed waveform (400 to 600 beats per minute), such that an irregular pulse occurs.

Atrial fibrillation (A-fib) is caused by a problem with the heart's electrical system. With A-fib, most electrical signals of the heart start from places other than the SA node. This makes the right atrium and left atrium beat very fast in an irregular rhythm. This irregular beating is called fibrillation. For some people, A-fib is constant. For others, A-fib comes and goes.

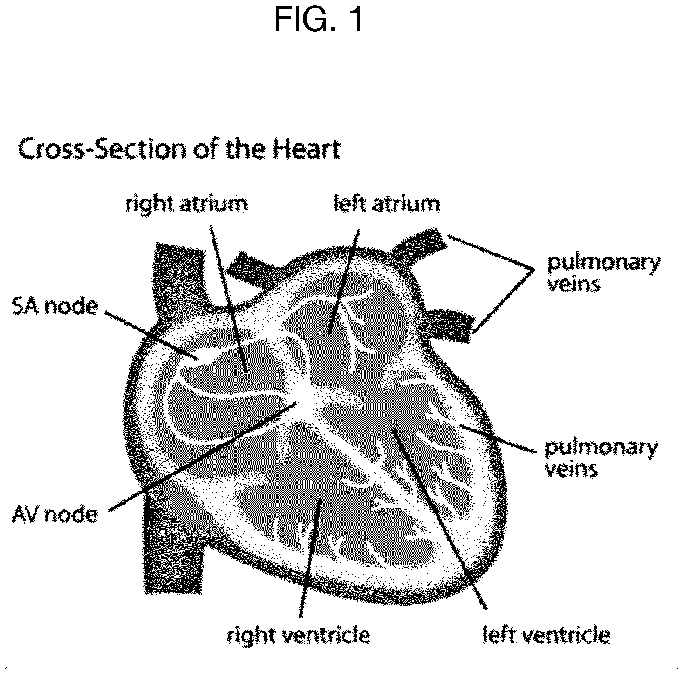

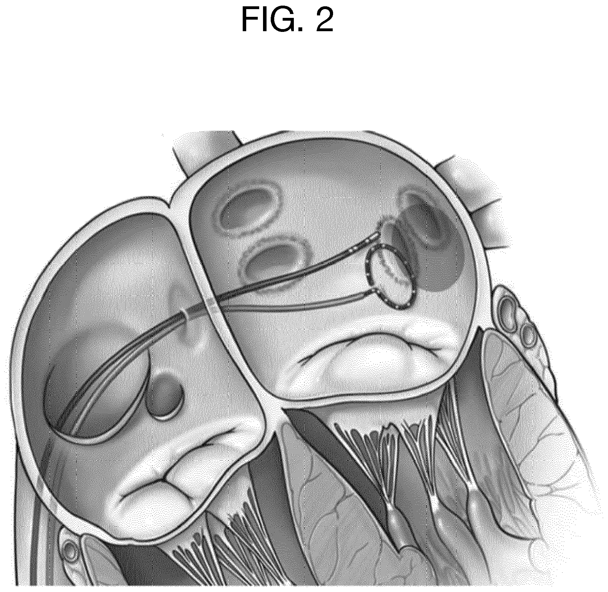

FIG. 1 is a cross-sectional view of the heart for describing the structure of the human heart, and FIG. 2 is a schematic diagram for describing radio frequency ablation (RFA) used for the treatment of atrial fibrillation.

Referring to FIGS. 1 and 2, RFA is one way to return the heartbeat back to its normal rhythm. RFA stops electrical signals that come from places other than the SA node. In RFA, thin wires are threaded into the heart through a vein in an arm or leg. One wire is used to find a problem area in the heart's electrical system. Then, heat is transferred through another wire. The heat destroys a small amount of tissue in the problem area and stops abnormal heart beats. Once all the problem areas are fixed, the wires are taken out.

### [Related Art Document]

### [Patent Document]

(Patent Document 0001) Korean Unexamined Patent Application Publication No. 10-2020-0060188 (Date of Publication: May 29, 2020)

US 2016/128767 A1 describes systems, devices and methods for modulating targeted nerve fibers (e.g., hepatic neuromodulation). The systems may be configured to access tortuous anatomy of or adjacent hepatic vasculature. The systems may be configured to target nerves within a wall of (e.g., within adventitia surrounding a lumen of) an artery or other blood vessel, such as the common hepatic artery.

WO 2019/148094 A1 discloses systems, devices, and methods for transvascular ablation of target tissue. The devices and methods may, in some examples, be used for splanchnic nerve ablation to increase splanchnic venous blood capacitance to treat at least one of heart failure and hypertension. For example, the devices disclosed herein may be advanced endovascularly to a target vessel in the region of a thoracic splanchnic nerve (TSN), such as a greater splanchnic nerve (GSN) or a TSN nerve root. Also disclosed are method of treating heart failure, such as HFpEF, by endovascularly ablating a thoracic splanchnic nerve to increase venous capacitance and reduce pulmonary blood pressure.

US 2019/076179 A1 describes an ablation apparatus for creating a lesion in target tissue, the ablation apparatus having an ablation shaft including a handle, a first portion, an ablation portion, distal tip, at least one ablation energy delivery lumen, at least one ablation energy return lumen, and a stylet lumen that extends substantially along a length of the ablation shaft from the handle to at least the ablation portion. The ablation apparatus also includes a stylet that is capable of being inserted into the stylet lumen where the stylet is made of a shape-memory material.

### SUMMARY OF THE DISCLOSURE

The present disclosure is directed to providing a RF ablation catheter for treatment of atrial fibrillation, which has a simple structure and is effective in treating atrial fibrillation of the heart, and a method for treatment of atrial fibrillation using the same.

The objectives of the present disclosure are not limited to those mentioned above, and other unmentioned objectives should be clearly understood by those of ordinary skill in the art to which the present disclosure pertains from the following description.

An exemplary embodiment of the present disclosure provides a RF ablation catheter for treatment of atrial fibrillation of the heart, the RF ablation catheter including: a body part which has one or more ablation electrodes formed on a distal part and which constitutes a catheter body made of a soft material; and a tapered tip which is connected to the body part and becomes thinner toward an end of the catheter, wherein a guidewire lumen is formed in the catheter to be connected to the tip and the body part, and wherein the guidewire lumen (26) is configured to insert a guidewire (10) therein.

According to an exemplary embodiment, the guidewire lumen may be formed from the end of the tip to a side hole formed in an intermediate portion of the catheter or may be formed from the end of the tip toward a proximal part of the catheter.

According to an exemplary embodiment, the tapered tip may have an atraumatic tip structure in which an edge of the end of the tip has a round shape so that, when the catheter is inserted along blood vessels, damage to the blood vessels does not occur.

According to an exemplary embodiment, the ablation electrode may be a coil-type ablation electrode which is wound in the shape of a coil around the catheter to improve flexibility.

According to an exemplary embodiment, an electrode wire connected to the ablation electrode may be a silver wire which is spirally wrapped around the catheter and covered with a material harmless to the human body.

According to an exemplary embodiment, in order to measure the temperature of the electrode, two strands of a thermocouple wire may be spirally wrapped around the catheter from the proximal part to the distal part while being wound together. More preferably, one strand of the thermocouple wire may be a nickel-chromium wire that is harmless to the human body, and the other strand of the thermocouple wire may be a nickel-alumel wire that is harmless to the human body.

According to the invention, one or more sensing electrodes which are spaced apart from the ablation electrode and configured to sense an electrical signal of the heart are formed on a surface of the body part.

According to the invention, a sensing electrode wire connected to the sensing electrode is a silver wire which is spirally wrapped around the catheter and covered with a material harmless to the human body.

According to an exemplary embodiment, the sensing electrode has a smaller width than the ablation electrode.

An exemplary embodiment of the present disclosure provides a method for treatment of atrial fibrillation of the heart, the method including: inserting a guidewire from the superior vena cava or inferior vena cava into the vein of Marshall via a coronary sinus; placing a RF ablation catheter, which has an ablation electrode formed on a distal part, in the vein of Marshall according to guidance of the guidewire; and performing RF ablation around the vein of Marshall after connecting the RF ablation catheter to a RF generator.

According to an embodiment, the RF ablation may be bipolar ablation in which a ground catheter is placed in the left atrium before performing the RF ablation and then ablation is performed between the RF ablation catheter and the ground catheter.

According to an exemplary embodiment, the method may further include, before or after the RF ablation, using a sensing electrode formed on the RF ablation catheter, using a separate electrophysiology (EP) catheter, or using the ground catheter to sense an abnormal electrical signal of the vein of Marshall.

### SUMMARY OF THE INVENTION

The invention is defined in independent claim 1. Particularly advantageous aspects of the invention are defined in the dependent claims. Any methods disclosed hereinafter do not form part of the invention as such but are described for illustrative purposes only, in the interest of fostering a better understanding of the invention.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a cross-sectional view of the heart for describing the structure of the human heart;
FIG. 2 is a view for describing a method for treatment of atrial fibrillation using conventional radio frequency ablation (RFA);
FIG. 3 is a schematic diagram of a cross-section of a catheter for treatment of atrial fibrillation according to an exemplary embodiment of the present disclosure;
FIGS. 4 to 6 illustrate various embodiments of the catheter for treatment of atrial fibrillation according to the present disclosure;
FIGS. 7 and 8 are schematic diagrams for describing radio frequency ablation (RFA), which is a method for treatment of atrial fibrillation using the catheter for treatment of atrial fibrillation, according to an exemplary embodiment of the present disclosure;
FIG. 9 is a flowchart of the method for treatment of atrial fibrillation according to FIGS. 7 and 8;
FIGS. 10 and 11 are schematic diagrams for describing radio frequency ablation (RFA), which is a method for treatment of atrial fibrillation using a catheter for treatment of atrial fibrillation, according to another exemplary embodiment of the present disclosure; and
FIG. 12 is a flowchart of the method for treatment of atrial fibrillation according to FIGS. 10 and 11.

### DETAILED DESCRIPTION OF EXEMPLARY EMBODIMENTS

The advantages, features, and methods of achieving the same of the present disclosure will become more apparent by referring to embodiments described in detail below with reference to the accompanying drawings. However, the present disclosure is not limited to the embodiments disclosed below and may be implemented in various different forms. The present embodiments make the disclosure of the present disclosure complete and are provided to completely inform those of ordinary skill in the art to which the present disclosure pertains of the scope of the present disclosure.

Details for embodying the present disclosure will be described in detail with reference to the accompanying drawings. The same reference numerals refer to the same elements regardless of the drawings, and the term "and/or" may refer to each mentioned item or any combination of one or more of the mentioned items.

The terms used herein are for describing the embodiments and are not intended to limit the present disclosure. In the present specification, a singular expression includes a plural expression unless the context clearly indicates otherwise. The terms "comprises" and/or "comprising" used herein do not preclude the presence or addition of one or more elements other than those mentioned.

Unless defined otherwise, all terms including technical or scientific terms used herein may have the same meaning as commonly understood by those of ordinary skill in the art to which the present disclosure pertains. Also, terms defined in commonly used dictionaries should not be construed in an idealized or overly formal sense unless expressly so defined herein.

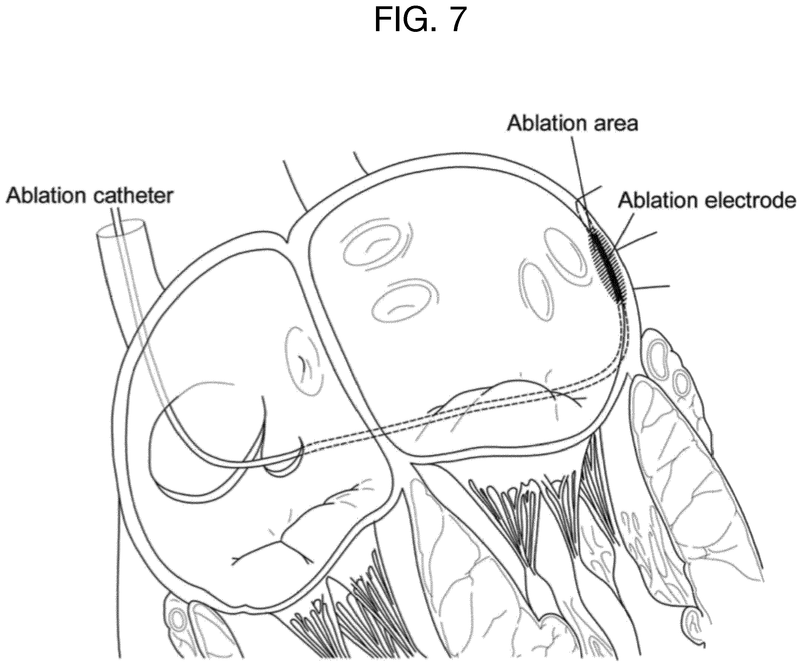

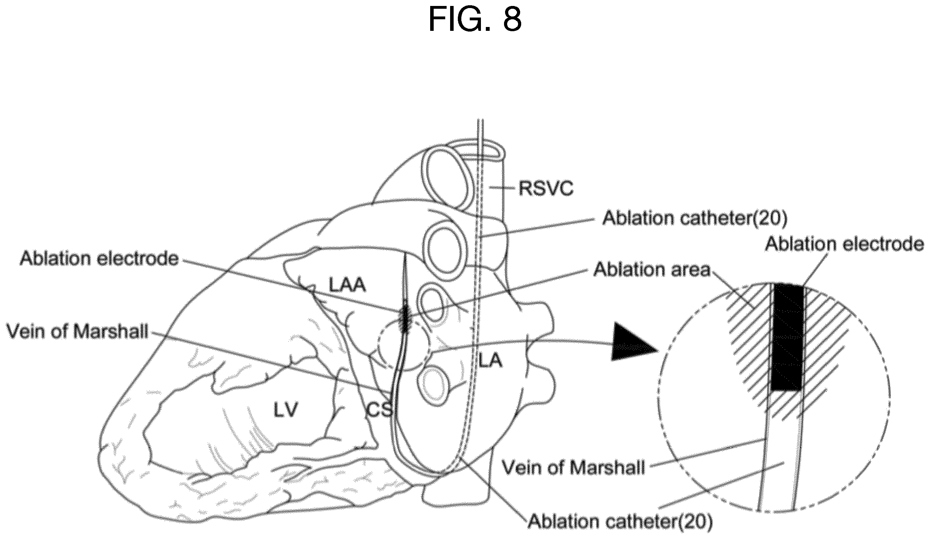

A method for treatment of atrial fibrillation according to the present disclosure is an indirect method of eliminating atrial fibrillation in which, by inserting a radio frequency ablation (RFA) catheter according to the present disclosure into the vein of Marshall (VOM) connected to the coronary sinus (CS) and then performing ablation, an abnormal electrical signal generated in the vicinity of the VOM is eliminated. Here, the VOM refers to the oblique vein of the left atrium that extends toward the left atrium (LA) before reaching the great cardiac vein from the CS of the posterior wall of the LA (see FIGS. 7 and 8).

That is, a method for treatment of atrial fibrillation according to the present disclosure is an indirect method of eliminating atrial fibrillation by eliminating an abnormal electrical signal generated in the vicinity of the VOM, in which a RFA catheter is inserted into the CS through the superior vena cava or inferior vena cava and then inserted into the VOM connected to the CS, an electrode wire is connected to a RF ablation generator at a proximal part of the RFA catheter, and then RF power is applied to the electrode wire so that RF ablation occurs at a RF ablation electrode disposed on a distal part of the RFA catheter.

Hereinafter, exemplary embodiments of the present disclosure will be described in detail with reference to the accompanying drawings.

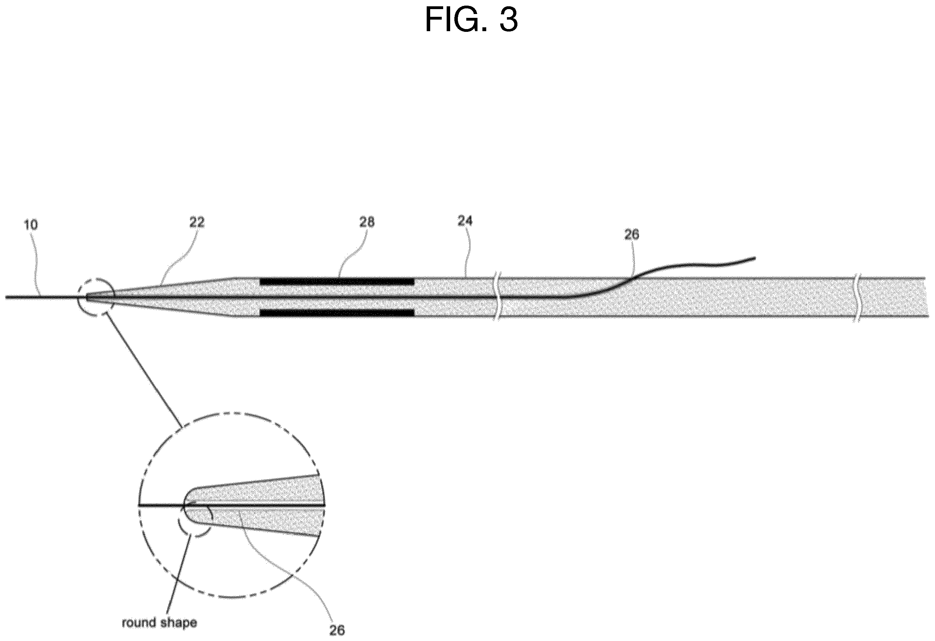

FIG. 3 is a schematic diagram of a cross-section of a catheter for treatment of atrial fibrillation according to an exemplary embodiment of the present disclosure.

Referring to FIG. 3, a catheter for treatment of atrial fibrillation according to the present disclosure is a RF ablation catheter that enters blood vessels (the CS and the vein of Marshall) along a guidewire 10. The catheter has a tapered tip 22 formed to narrow toward an end of a distal part and has a RF ablation electrode 28 for RF ablation formed on a surface of a body part 24.

Preferably, the catheter according to the present disclosure has a thickness of around 4 to 6 Fr (about 1.32 to 2 mm). More preferably, the catheter has a diameter of about 5 Fr. A portion of the catheter where the RF ablation electrode is disposed should be inserted into the VOM of the heart, and since a typical lumen diameter of the human VOM is about 1.3 mm in average, the catheter preferably has the thickness of around 4 to 6 Fr (about 1.32 to 2 mm) and more preferably has the diameter of about 5 Fr.

The catheter according to the present disclosure has the tapered tip 22 formed to narrow toward an end of the catheter. The tapered tip 22 has a length in a range of about 5 to 20 mm (preferably, about 10 mm), and a sharp end of the tapered tip 22 has a thickness in a range of about 1.2 to 1.4 Fr. The opposite side of the sharp end has the same thickness as the body part (preferably, a thickness in a range of 4 to 6 Fr).

The catheter according to the present disclosure has an atraumatic tip structure that does not cause damage to blood vessels when the catheter is inserted along blood vessels of the CS and VOM. That is, in order to prevent damage to blood vessels into which the catheter is inserted, an edge of the end of the tip has a round shape as illustrated in FIG. 3.

Since the catheter according to the present disclosure enters the VOM along the guidewire, a guidewire lumen 26 into which the guidewire is inserted is formed in the catheter.

In FIG. 3, the catheter according to the present disclosure is illustrated as a monorail type RF ablation catheter in which the guidewire 10 that enters the catheter through the tip 22, which is the end of the distal part of the catheter, exits the catheter through a side hole formed in a side surface of an intermediate portion of the catheter, but the catheter according to the present disclosure is not necessarily limited thereto. The catheter according to the present disclosure may also be an over-the-wire type RF ablation catheter in which the guidewire is connected from the end (the end of the distal part), which is the tip portion, to an end of a proximal part.

However, the monorail type RF ablation catheter is more preferable than the over-the-wire type RF ablation catheter. This is because the monorail type RF ablation catheter allows an operator to perform treatment more conveniently when performing treatment using the catheter according to the present disclosure. In the case of the monorail type RF ablation catheter, since the guidewire 10 exits the catheter through the side surface of the intermediate portion of the catheter, the operator can easily take out the guidewire from the catheter. Thus, the operator can easily handle the catheter and more conveniently perform treatment.

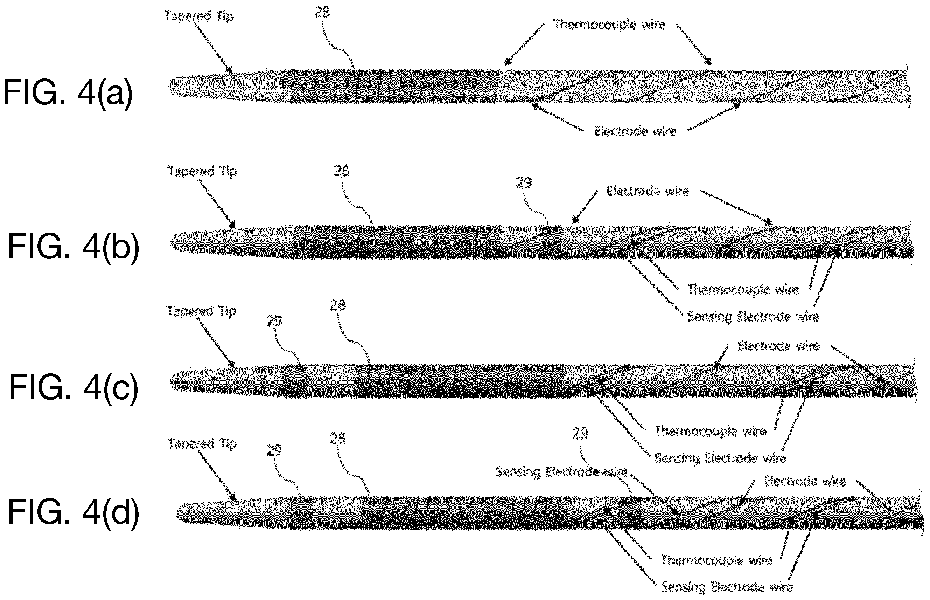

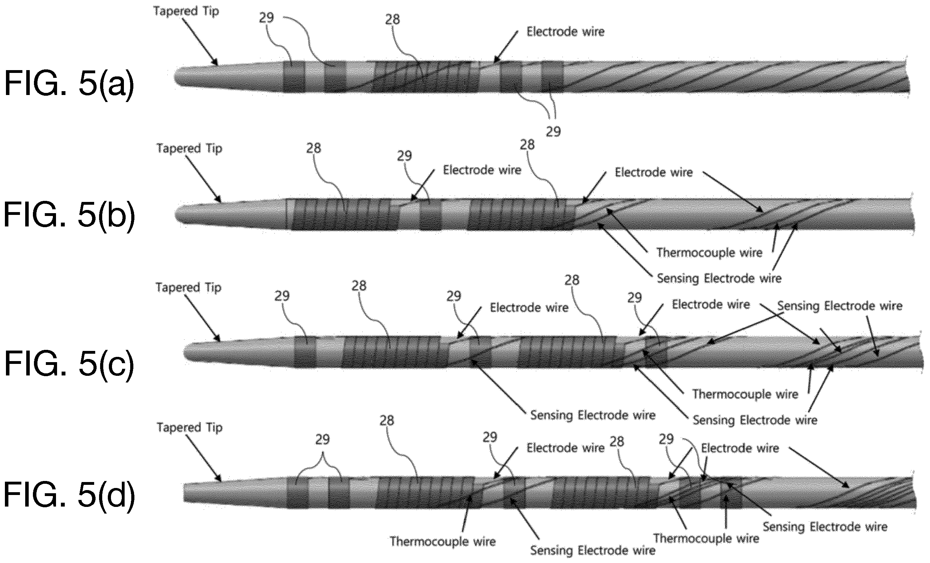

Power for RF ablation is supplied to the RF ablation electrode 28 through an electrode wire (not illustrated in FIG. 3 and illustrated in FIGS. 4 and 5) formed inside the catheter or on a surface thereof.

FIGS. 4 to 6 illustrate various embodiments of the catheter for treatment of atrial fibrillation according to the present disclosure.

FIG. 4A illustrates a case in which a single RF ablation electrode 28 is formed, FIGS. 4B and 4C illustrate cases in which a single RF ablation electrode 28 and a single sensing electrode 29 are formed, and FIG. 4D illustrates a case in which a single RF ablation electrode 28 and two sensing electrodes 29 are formed, the sensing electrode 29 each being formed to the left and right of the RF ablation electrode 28.

FIG. 5A illustrates a case in which a single RF ablation electrode 28 and a total of four sensing electrodes 29 are formed, two sensing electrodes 29 each being formed to the left and right of the RF ablation electrode 28, FIG. 5B illustrates a case in which two RF ablation electrodes 28 are formed and a single sensing electrode 29 is formed therebetween, FIG. 5C illustrates a case in which two RF ablation electrodes 28 and three sensing electrodes 29 are alternately formed, and FIG. 5D illustrates a case in which two RF ablation electrodes 28 and a total of five sensing electrodes 29 are formed, wherein two sensing electrodes 29 are formed to the left of the left RF ablation electrode 28, a single sensing electrode 29 is formed between the two RF ablation electrodes 28, and two sensing electrodes 29 are formed to the right of the right RF ablation electrode 28.

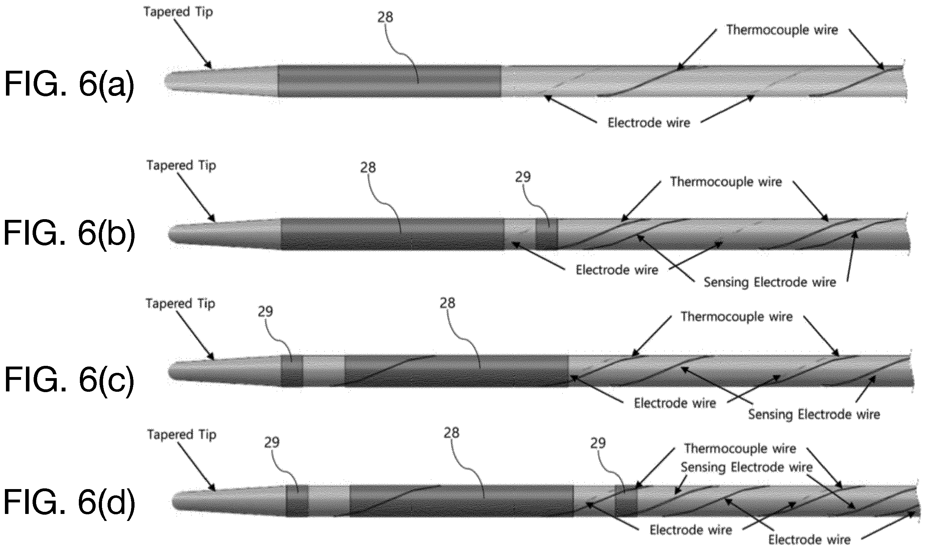

FIG. 6 illustrates the same cases as in FIG. 4 except that FIG. 6 illustrates ring-shaped RF ablation electrodes while FIGS. 4 and 5 illustrate coil-type RF ablation electrodes that are formed by the RF ablation electrode 28 being wound in the form of a coil.

First, referring to FIG. 4, the coil-type RF ablation electrodes that are formed by the RF ablation electrode 28 being wound in the form of a coil are illustrated.

The RF ablation electrode 28 may be in the form of a coil (FIGS. 4 and 5) or have a ring shape (FIG. 6), but the RF ablation electrode 28 being in the form of a coil as illustrated in FIGS. 4 and 5 is preferable.

The RF ablation electrode is wound in the form of a coil as illustrated in FIGS. 4 and 5 in order to maximize flexibility, that is, to allow the catheter to more easily follow the curved blood vessels along the guidewire. A portion where the RF ablation electrode of the catheter is finally placed is located in the VOM of the heart. To this end, the catheter is inserted along the curved blood vessels (CS and VOM), and since the catheter has a characteristic of being flexible, the catheter may be more easily inserted into the VOM.

The RF ablation electrode may have various widths but, preferably, may have a width in a range of about 5 to 20 mm. As illustrated, one or more RF ablation electrodes are installed. That is, one or more RF ablation electrodes with different widths may be used in a single catheter. Electrodes with a larger width may be used in a case in which the number of electrodes is small, and electrodes with a smaller width may be used in a case in which the number of electrodes is large.

Even when a plurality of electrodes are formed, since a range of ablation may be disconnected when intervals between the electrodes is excessively wide, preferably, the intervals between the RF ablation electrodes is within 2 mm.

Even in a case in which a plurality of RF ablation electrodes are used, the electrodes have the same polarity. Monopolar electrodes whose +/-, phase, and size are the same may be simultaneously used. Even when a single electrode having a large width is formed using a plurality of monopolar electrodes having a smaller width, the same ablation effect may be achieved.

RF energy for performing RF ablation is supplied to the RF ablation electrode 28 through an electrode wire that is spirally wrapped around a surface of the catheter made of a soft plastic material. In the present disclosure, due to spirally forming the electrode wire around the surface of the catheter, straightness of the catheter may be improved and pushability thereof may be improved.

The electrode wire is a wire configured to supply RF energy. Preferably, a silver wire covered with a sheath is used as the electrode wire. The silver wire is insulated (covered) with a plastic material harmless to the human body. Since the electrode wire has a structure surrounding the surface of the catheter, in a case in which the sheath of the electrode wire comes off when the catheter is inserted into the vena cava, CS, and VOM, the electrode wire comes in contact with a tissue. Thus, materials such as copper that are harmful to the human body cannot be used. Therefore, preferably, a silver wire harmless to the human body is used as the electrode wire.

In addition to the silver wire which is the electrode wire, a thermocouple wire is spirally wrapped around the surface of the catheter while being spaced apart from the electrode wire. The thermocouple wire is also covered with a harmless plastic material. The thermocouple wire for measuring a temperature of the distal part of the catheter is connected.

The thermocouple wire is for measuring the temperature around the electrode. That is, the thermocouple wire is a wire used as a thermoelectric thermometer. The thermocouple wire has a form in which two wires covered with a sheath are wound together and is spirally wrapped around the surface of the catheter while being spaced apart from the electrode wire (silver wire). The thermocouple wire is entirely covered with the sheath except for ends of the two wires so that the two wires are connected to each other and used as a thermoelectric thermometer. The thermoelectric thermometer may be formed inside the catheter, approximately at an intermediate point of the RF ablation electrode.

Various thermocouple wires are in use nowadays, but in the present disclosure, since the thermocouple wire has a form wrapped around the surface of the catheter inserted into the human body, preferably, nickel-chromium or nickel-alumel is used for the thermocouple wire so that it is harmless to the human body even when the sheath of the thermocouple wire comes off. That is, one strand of the thermocouple wire is a nickel-chromium wire harmless to the human body, and the other strand of the thermocouple wire is a nickel-alumel wire harmless to the human body.

Since the thermocouple wire of the present disclosure is also spirally wrapped around the surface of the catheter, it is possible to obtain advantages in that straightness of the catheter is improved and pushability thereof is improved.

Meanwhile, one or more sensing electrodes 29 are formed on the surface of the catheter while being spaced apart from the RF ablation electrode.

The sensing electrode corresponds to a sensor for sensing an electrical signal around the VOM of the heart to allow a therapeutic effect to be checked immediately after RF ablation therapy. The sensing electrode is connected to the outside by a sensing electrode wire that is separate from the thermocouple wire or electrode wire. As many sensing electrode wires as the number of sensing electrodes are connected.

Since the sensing electrode is for sensing an electrical signal of the heart, the width of the sensing electrode is less than the width of the RF ablation electrode. While the width of the RF ablation electrode is in a range of about 5 to 20 mm, the sensing electrode preferably has a width in a range of 1 to 5 mm.

The sensing electrode wire is also spirally wrapped around the surface of the catheter so that the straightness of the catheter is improved and pushability thereof is improved.

Due to the reason described above, a silver wire insulated (covered) with a plastic material harmless to the human body is also used as the sensing electrode wire.

In FIGS. 4 to 6, all of the electrode wire, the sensing electrode wire, and the thermocouple wire are illustrated as being visible on the surface of the catheter, but of course, the entire catheter may be surrounded by a covering material such that the electrode wire, the sensing electrode wire, and the thermocouple wire are not visible from the outer surface of the catheter.

FIG. 6 illustrates a case in which a ring-shaped RF ablation electrode is used.

In the case in which the ring-shaped RF ablation electrode is used, flexibility may be decreased as compared to when the coil-type RF ablation electrode illustrated in FIGS. 4 and 5 is used. In a case in which the ring-shaped electrode is used, flexibility is inversely proportional to the width of the ring shape. That is, the larger the width of the electrode, the lower the flexibility. Therefore, rather than using a single ring-shaped electrode having a wide width, a plurality of ring-shaped electrodes having a small width (about 2mm) may be disposed in parallel for use. Of course, in this case, the plurality of electrodes are monopolar electrodes.

Various embodiments are illustrated in FIGS. 4 to 6, but the present disclosure is not necessarily limited thereto, and of course, the number of RF ablation electrodes and sensing electrodes and the positions thereof may be changed in any way by a user as necessary.

FIGS. 4 to 6 illustrate cases in which a cooling tube is not present in the catheter, but a cooling tube for injecting a coolant from the outside to address problems that occur during ablation may be formed in the catheter as necessary.

Next, a method of performing treatment of atrial fibrillation using a catheter for treatment of atrial fibrillation according to the present disclosure will be described.

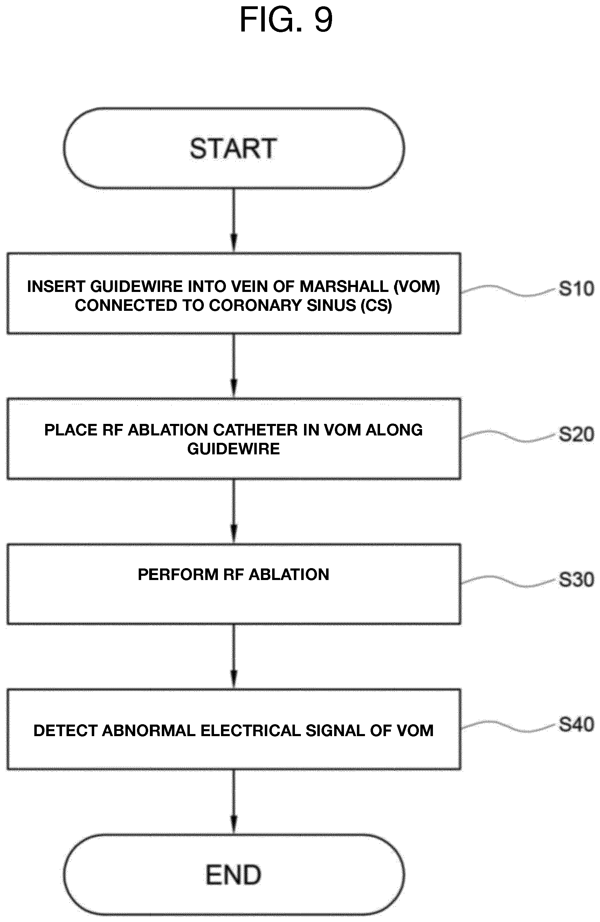

FIGS. 7 and 8 are schematic diagrams for describing radio frequency ablation (RFA), which is a method for treatment of atrial fibrillation using the catheter for treatment of atrial fibrillation, according to an exemplary embodiment of the present disclosure, and FIG. 9 is a flowchart of the method for treatment of atrial fibrillation according to an exemplary embodiment of the present disclosure.

First, a guidewire is inserted into the VOM connected to the CS (S10).

In the present disclosure, in order to access the VOM, the CS having an opening located in the right atrium is used. Access to the CS is made using a neck vein or a femoral vein or is made using a guiding catheter via the superior vena cava or inferior vena cava.

As the guiding catheter, a balloon-tipped guiding catheter and/or a balloon-tipped dual lumen microcatheter is used. The guiding catheter is a catheter for guiding the guidewire to a desired position.

Upon accessing the CS, the guiding catheter is placed at the distal part of the CS, and a pressurized venogram is performed through the guiding catheter. A balloon-tipped guiding catheter is ideal for a pressurized venogram.

After a septal vein venogram is performed through the pressurized venogram to check the location and size of the VOM, a percutaneous transluminal coronary angioplasty (PTCA) guidewire having a diameter of about 0.014" is inserted into the VOM which is the final target connected to the septal vein.

For the PTCA guidewire, imaging guidance in which real-time imaging equipment for an echocardiogram or the like is utilized to notify whether the wire reaches the target site enables more precise treatment. In order to more precisely guide the orientation of the PTCA guidewire, a dual lumen microcatheter may be used to precisely guide the wire to the target site.

In other words, in order to place the guidewire in the VOM, (1) a pressurized venogram with a balloon-tipped guiding catheter and/or (2) a dual lumen microcatheter may be used.

A pressurized venogram is used to make the septal vein more visible. To this end, a balloon-tipped guiding catheter may be used as an auxiliary means.

Also, a dual lumen microcatheter or the like may be used as an auxiliary means to place the guidewire in a desired direction in the interventricular septum. The dual lumen microcatheter is a catheter in which two lumens are formed to insert guidewires. A first guidewire is placed in the septal vein through a first lumen of the dual lumen microcatheter, and then a second guidewire is inserted through a second lumen of the dual lumen microcatheter. The second guidewire may be moved in a different direction from the first guidewire. In this way, the dual lumen microcatheter is an auxiliary catheter for treatment that is very useful in allowing the guidewire to reach a target site.

In this way, the balloon-tipped catheter and dual lumen catheter serve as guiding catheters and guide the guidewire to the VOM.

The guidewire enters the right atrium (RA) via the superior vena cava or inferior vena cava and then enters the CS from the RA. Then, the guidewire enters the VOM (the oblique vein of the LA), which extends toward the LA before reaching the great cardiac vein from the CS, and is placed therein.

In this way, according to guidance of the guiding catheter, the guidewire is placed in the VOM connected to the CS.

Next, a RF ablation catheter for treatment of atrial fibrillation is placed in the VOM according to guidance of the guidewire (S20).

After inserting the guidewire into the guidewire lumen formed in the RF ablation catheter according to the present disclosure, the catheter is pushed into the heart so that a RF ablation electrode of the RF ablation catheter is located in the VOM.

Since the RF ablation catheter according to the present disclosure has been described in detail above, detailed description thereof will be omitted.

Next, after placing the RF ablation catheter in the VOM, RF ablation is performed (S30).

The RF ablation catheter is connected to a RF generator configured to generate RF energy at a proximal part, and then RF ablation is performed.

As illustrated in FIGS. 7 to 9, in a case in which only the RF ablation catheter is used without using a ground catheter (that is, monopolar ablation is performed), an ablation area is formed in the shape of a tower around the RF ablation electrode.

After the ablation is completed, an abnormal electrical signal (abnormal signal) generated in the VOM is checked (S40).

After the RF ablation is completed, a sensing electrode formed on the catheter according to the present disclosure is used, a separate electrophysiology (EP) catheter is used, or a ground catheter is used in order to check an abnormal electrical signal generated in the VOM.

In a case in which it is unclear as to whether an abnormal electrical signal generated in the VOM is eliminated, RF ablation may be performed again.

After a desired therapeutic effect is obtained by performing RF ablation, the device is entirely removed to complete the RF ablation for treatment of atrial fibrillation.

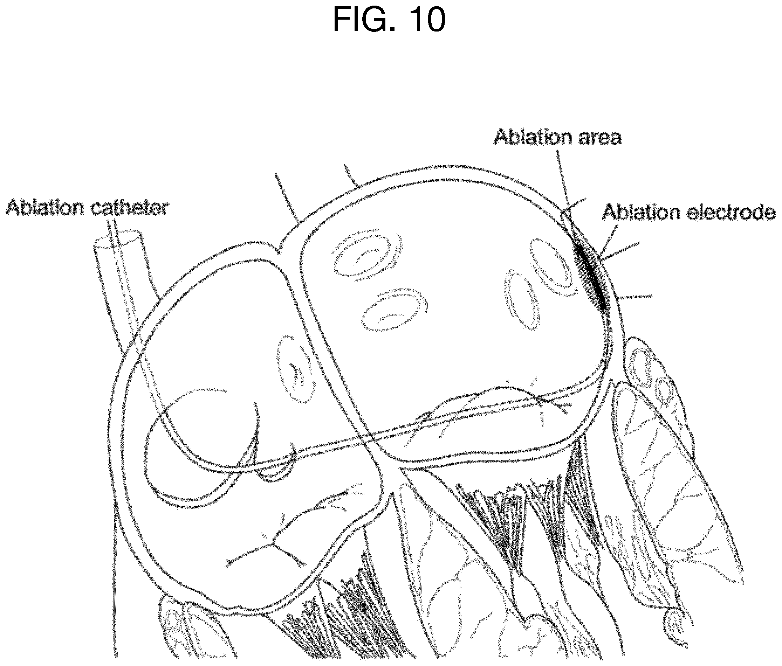

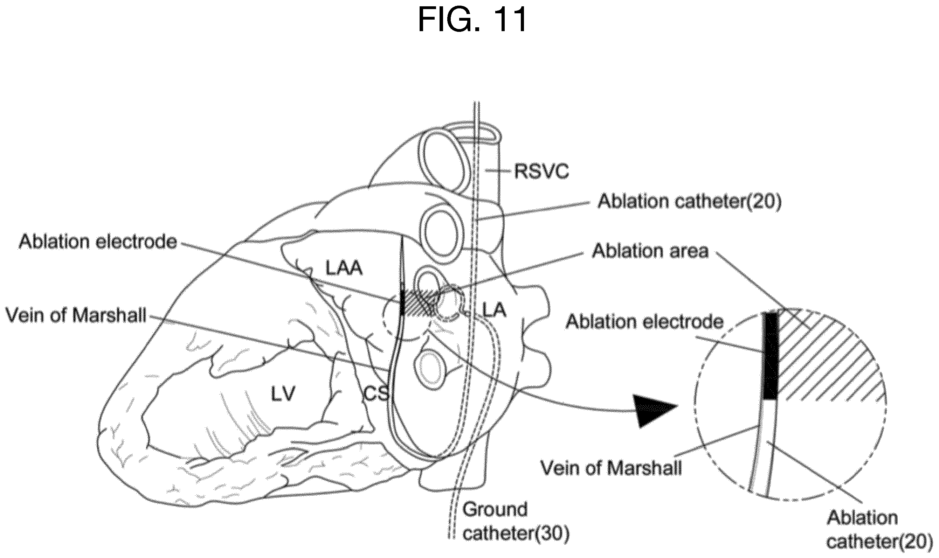

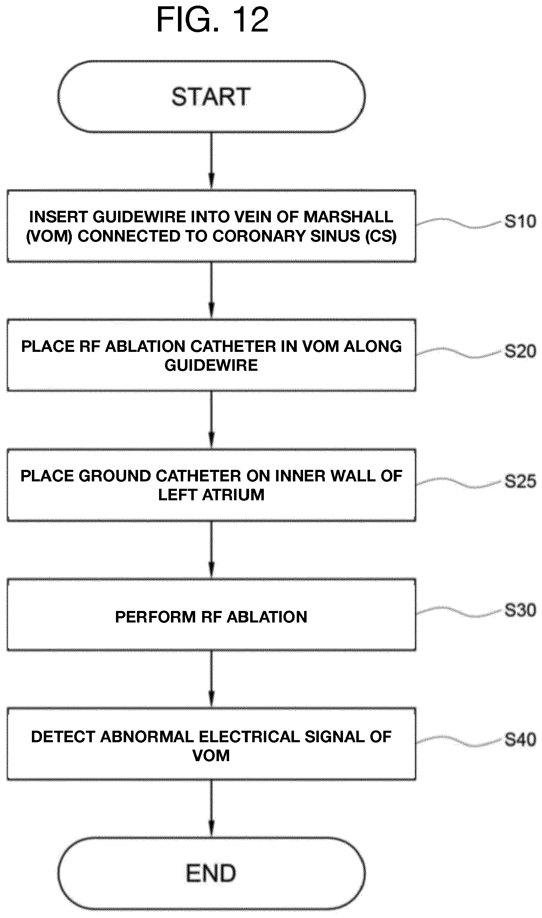

Meanwhile, FIGS. 10 and 11 are schematic diagrams for describing radio frequency ablation (RFA), which is a method for treatment of atrial fibrillation using a catheter for treatment of atrial fibrillation, according to another exemplary embodiment of the present disclosure, and FIG. 12 is a flowchart of the method for treatment of atrial fibrillation according thereto.

While the treatment method illustrated in FIGS. 7, 8, and 9 is a method of performing monopolar RF ablation in which only the RF ablation catheter is used, the treatment method illustrated in FIGS. 10, 11, and 12 is a method of performing bipolar RF ablation in which a ground catheter is also used.

Inserting a guidewire into the VOM connected to the CS (S10) and placing a RF ablation catheter for treatment of atrial fibrillation in the VOM according to guidance of the guidewire (S20) are the same as those described above with reference to FIGS. 7, 8, and 9.

As illustrated in FIGS. 10, 11, and 12, for bipolar RF ablation, a ground catheter is placed on an inner wall of the LA (S25).

A ground catheter 30 is brought into the RA through the superior vena cava or inferior vena cava (mostly using the inferior vena cava) and then brought into the LA by passing through atrial septal muscle between the RA and LA. Then, a site where an electrical signal of the heart irregularly flows on the inner wall of the LA is identified, and then the ground catheter is placed at the identified site. The ground catheter is mostly placed at an opening of the pulmonary vein (PV).

Next, RF ablation is performed (S30).

The RF ablation catheter is connected to a RF generator configured to generate RF energy, and then RF ablation is performed.

As illustrated in FIGS. 10 to 12, in the case in which the ground catheter 30 is used, ablation is performed between the ground catheter and the RF ablation catheter. That is, in the case of bipolar RF ablation in which the ground catheter is used, an exchange of RF energy occurs between the ground catheter and the RF ablation catheter, and thus ablation occurs in an area between the ground catheter and the RF ablation catheter.

Meanwhile, the case in which the RF ablation catheter is placed in the VOM and the ground catheter is placed in the LA has been illustrated and described above, but the locations of the RF ablation catheter and the ground catheter may also be reversed. That is, among the two catheters placed inside and outside the LA, any one may serve as the RF ablation catheter while the other one serves as the ground catheter, and such roles of the catheters may be determined according to convenience of the operator.

Other details of the bipolar RF ablation method illustrated in FIGS. 10 to 12 are the same as those of the monopolar RF ablation method illustrated in FIGS. 7 to 9 and thus will be omitted.

As described above, a RF ablation catheter for treatment of atrial fibrillation according to the present disclosure is easy to insert along blood vessels due to having a simple structure, a tapered end, and a round shape and is easy to insert along a guidewire due to having a guidewire lumen formed therein.

Also, a method for treatment of atrial fibrillation according to the present disclosure has an advantage in that, since ablation is performed after a RF ablation catheter is inserted through the vein of Marshall, an ablation area is expanded, and thus atrial fibrillation can be effectively treated.

The embodiments of the present disclosure have been described above with reference to the accompanying drawings, but those of ordinary skill in the art to which the present disclosure pertains should understand that the present disclosure may be embodied in other specific forms.

Therefore, the embodiments described above should be understood as being illustrative, instead of limiting, in all aspects.

## Claims

1. A catheter for treatment of atrial fibrillation of the heart, the catheter comprising:
a body part (24) which has one or more ablation electrodes (28) formed on a distal part and which constitutes a catheter body made of a soft material; and
a tapered tip (22) which is connected to the body part (24) and becomes thinner toward an end of the catheter,
wherein a guidewire lumen (26) is formed in the catheter and fluidly connects to the tip (22) and the outer surface of the body part (24) proximal to the one or more ablation electrodes (28), and
wherein the guidewire lumen (26) is configured to insert a guidewire (10) therein,
**characterized in that**
one or more sensing electrodes (29) which are spaced apart from the ablation electrode (28) and configured to sense an electrical signal of the heart are formed on a surface of the body part (24), and
a sensing electrode wire connected to the sensing electrode (29) is a silver wire which is spirally wrapped around the catheter and covered with a material harmless to the human body.

2. The catheter of claim 1, wherein the guidewire lumen (26) is formed from the end of the tip (22) to a side hole formed in a side surface of an intermediate portion of the catheter.

3. The catheter of claim 1, wherein the guidewire lumen (26) is connected from the end of the tip (22) toward a proximal part of the catheter.

4. The catheter of any one of claims 1 to 3, wherein the tapered tip (22) has an atraumatic tip structure in which an edge of the end of the tip (22) has a round shape so that, when the catheter is inserted along a blood vessel, damage to the blood vessel does not occur.

5. The catheter of any one of claims 1 to 4, wherein the ablation electrode (28) is a coil-type ablation electrode which is wound in the shape of a coil around the catheter to improve flexibility.

6. The catheter of any one of claims 1 to 5, wherein an electrode wire connected to the ablation electrode (28) is a silver wire which is spirally wrapped around the catheter and covered with a material harmless to the human body.

7. The catheter of any one of claims 1 to 6, wherein, in order to measure the temperature of the electrode, two strands of a thermocouple wire are spirally wrapped around the catheter from the proximal part to the distal part while being wound together.

8. The catheter of claim 5, wherein one strand of the thermocouple wire is a nickel-chromium wire that is harmless to the human body, and the other strand of the thermocouple wire is a nickel-alumel wire that is harmless to the human body.

9. The catheter of claim 1, wherein the sensing electrode (29) has a smaller width than the ablation electrode (28).

## Patentansprüche

1. Katheter zur Behandlung von Vorhofflimmern des Herzens, wobei der Katheter aufweist:
ein Körperteil (24), das eine oder mehr Ablationselektroden (28) aufweist, die an einem distalen Teil ausgebildet sind, und das einen aus einem weichen Material bestehenden Katheterkörper bildet; und
eine zulaufende Spitze (22), die mit dem Körperteil (24) verbunden ist und in Richtung eines Endes des Katheters dünner wird;
wobei ein Führungsdrahtlumen (26) in dem Katheter gebildet ist und fluidisch mit der Spitze (22) und der Außenfläche des Körperteils (24) proximal der einen oder den mehreren Ablationselektroden (28) verbunden ist, und
wobei das Führungsdrahtlumen (26) zum Einführen eines Führungsdrahts (10) in dieses ausgebildet ist,
**dadurch gekennzeichnet,**
**dass** eine oder mehrere Sensierelektroden (29), welche von der Ablationselektrode (28) beabstandet sind und zum Erfassen eines elektrischen Signals des Herzens ausgebildet sind, auf einer Fläche des Körperteils (24) gebildet sind, und
**dass** ein mit der Sensierelektrode (29) verbundener Sensierelektrodendraht ein Silberdraht ist, der spiralförmig um den Katheter gewickelt ist und mit einem für den Menschen ungefährlichen Material bedeckt ist.

2. Katheter nach Anspruch 1, bei welchem das Führungsdrahtlumen (26) von dem Ende der Spitze (22) bis zu einem in einer Seitenfläche eines Mittelbereichs des Katheters gebildeten Seitenlochs ausgebildet ist.

3. Katheter nach Anspruch 1, bei welcher das Führungsdrahtlumen (26) von dem Ende der Spitze (22) in Richtung eines proximalen Teils des Katheters verbunden ist.

4. Katheter nach einem der Ansprüche 1 bis 3, bei welchem die zulaufende Spitze (22) eine atraumatische Spitzenstruktur aufweist, bei welcher ein Rand des Endes der Spitze (22) eine runde Form aufweist, so dass keine Beschädigung eines Blutgefäßes auftritt, wenn der Katheter entlang eines Blutgefäßes eingeführt wird.

5. Katheter nach einem der Ansprüche 1 bis 4, bei welchem die Ablationselektrode (28) eine Ablationselektrode vom Spulentyp ist, die zur Verbesserung der Flexibilität in Form einer Spule um den Katheter gewunden ist.

6. Katheter nach einem der Ansprüche 1 bis 5, bei welchem ein mit der Ablationselektrode (28) verbundener Elektrodendraht ein Silberdraht ist, der spiralförmig um den Katheter gewickelt ist und mit einem für den Menschen ungefährlichen Material bedeckt ist.

7. Katheter nach einem der Ansprüche 1 bis 6, bei welchem zum Messen der Temperatur der Elektrode zwei Litzen eines Thermoelementdrahts von dem proximalen Teil zum distalen Teil spiralförmig um den Katheter gewunden sind, wobei sie zusammen gewunden sind.

8. Katheter nach Anspruch 5, bei welchem eine Litze des Thermoelementdrahts ein Nickel-Chrom-Draht ist, welcher für den menschlichen Körper ungefährlich ist, und die andere Litze des Thermoelementdrahts ein Nickel-Alumel-Draht ist, welcher für den menschlichen Körper ungefährlich ist.

9. Katheter nach Anspruch 1, bei welchem die Sensierelektrode (29) eine geringere Breite als die Ablationselektrode (28) aufweist.

## Revendications

1. Cathéter pour le traitement d'une fibrillation auriculaire du coeur, le cathéter comprenant :
une partie de corps (24) qui comporte une ou plusieurs électrodes d'ablation (28) formées sur une partie distale et qui constitue un corps de cathéter fait d'un matériau souple ; et
une pointe (22) effilée qui est raccordée à la partie de corps (24) et qui s'amincit vers une extrémité du cathéter,
dans lequel une lumière de fil-guide (26) est formée dans le cathéter et se raccorde de manière fluide à la pointe (22) et à la surface externe de la partie de corps (24) proximale aux une ou plusieurs électrodes d'ablation (28), et
dans lequel la lumière de fil-guide (26) est configurée pour y insérer un fil-guide (10),
**caractérisé en ce que**
une ou plusieurs électrodes de détection (29) qui sont espacées de l'électrode d'ablation (28) et configurées pour détecter un signal électrique du coeur sont formées sur une surface de la partie de corps (24), et
un fil d'électrode de détection raccordé à l'électrode de détection (29) est un fil d'argent qui est enroulé en spirale autour du cathéter et recouvert d'un matériau inoffensif pour le corps humain.

2. Cathéter selon la revendication 1, dans lequel la lumière de fil-guide (26) est formée depuis l'extrémité de la pointe (22) jusqu'à un trou latéral formé dans une surface latérale d'une portion intermédiaire du cathéter.

3. Cathéter selon la revendication 1, dans lequel la lumière de fil-guide (26) est raccordée depuis l'extrémité de la pointe (22) vers une partie proximale du cathéter.

4. Cathéter selon l'une quelconque des revendications 1 à 3, dans lequel la pointe (22) effilée a une structure de pointe atraumatique dans laquelle un bord de l'extrémité de la pointe (22) a une forme ronde de sorte que, lorsque le cathéter est inséré le long d'un vaisseau sanguin, le vaisseau sanguin n'est pas endommagé.

5. Cathéter selon l'une quelconque des revendications 1 à 4, dans lequel l'électrode d'ablation (28) est une électrode d'ablation de type bobine qui est enroulée sous la forme d'une bobine autour du cathéter pour améliorer la flexibilité.

6. Cathéter selon l'une quelconque des revendications 1 à 5, dans lequel un fil d'électrode raccordé à l'électrode d'ablation (28) est un fil d'argent qui est enroulé en spirale autour du cathéter et recouvert d'un matériau inoffensif pour le corps humain.

7. Cathéter selon l'une quelconque des revendications 1 à 6, dans lequel, afin de mesurer la température de l'électrode, deux brins d'un fil de thermocouple sont enroulés en spirale autour du cathéter, de la partie proximale à la partie distale, tout en étant enroulés ensemble.

8. Cathéter selon la revendication 5, dans lequel un brin du fil de thermocouple est un fil de nickel-chrome qui est inoffensif pour le corps humain, et l'autre brin du fil de thermocouple est un fil de nickel-alumel qui est inoffensif pour le corps humain.

9. Cathéter selon la revendication 1, dans lequel l'électrode de détection (29) a une largeur plus petite que l'électrode d'ablation (28).
